# EUROPEAN PATENT SPECIFICATION

(11) **EP 3 849 961 B1**
(45) Date of publication and mention of the grant of the patent: **30.08.2023**
(21) Application number: 19763009.8
(22) Date of filing: 10.09.2019
(51) Int. Cl.: C07C 201/02, C07C 203/04, A61K 31/04, A61K 31/045, A23K 20/105, A23K 50/10

(54) **PROCESS FOR THE MANUFACTURE OF OMEGA NITROOXY-1-ALKANOLS**
VERFAHREN ZUR HERSTELLUNG VON OMEGA-NITROOXY-1-ALKANOLEN
PROCÉDÉ DE FABRICATION D'OMÉGA NITROOXY-1-ALCANOLES

(30) Priority: 14.09.2018 EP 18194566
(43) Date of publication of application: 21.07.2021
(73) Proprietor: DSM IP Assets B.V., 6411 TE Heerlen (NL)
(72) Inventor: EGGEL, Michael, 4303 Kaiseraugst (CH); KAPFERER, Peter, 4303 Kaiseraugst (CH); LUETHI, Erika, 4303 Kaiseraugst (CH)
(74) Representative: Berg, Katja
(86) International application number: PCT/EP2019/074072
(87) International publication number: WO 2020/053194

(56) References cited:
- EP-A1- 1 560 804
- WO-A1-2012/084629
- FR-A- 1 357 403

## Description

The invention relates to a safe and efficient process for the manufacture of ω-nitrooxy-C₃₋₁₀alkane-1-ols.

Global temperature is increasing, a process referred to as global warming or climate change. One of the main focuses to reduce this warming effect is to reduce the amount of greenhouse gases emitted into the atmosphere. Greenhouse gases are emitted from several different sources, both natural and anthropogenic; however, the two sources with the biggest impact are the agricultural and fossil fuel industries. Within agriculture, ruminants and in particular cattle are the major contributors to the biogenic methane formation, and it has been estimated that the prevention of methane formation from ruminants would almost stabilize atmospheric methane concentrations. 3-Nitrooxypropanol (3-NOP, also known as 3-nitrooxy-propan-1-ol or 1,3-propanediol mononitrate) has been reported to be highly efficient in reducing the formation of methane in ruminants without affecting microbial fermentation in a way that would be detrimental to the host animal (WO-2012/084629).

The mononitration of polyols is generally poorly selective and quickly leads to formation of di- or polynitrated alcohols since the kinetics are not easy to control. Moreover, by carrying out the same reaction on short chain alkanediols strong decomposition reactions are easily triggered.

Thus, WO-2012/084629 discloses the preparation of 3-nitrooxypropanol by reacting 3-bromopropanol in acetonitrile with silver nitrate, a process which is, however, not economical in industrial scale production.

Zikas et al (Bioorganic & Medicinal Chemistry, 13 (2005) 6485-6492) discloses the mononitration of alkanediols with acetyl nitrate (generated from nitric acid, acetic acid and acetic anhydride) at room temperature in the presence of ethyl acetate. Next to the necessity of an organic solvent, another drawback of this process is the use of acetyl nitrate which, even though an excellent nitration agent, is very explosive and thus not feasible for industrial scale production.

WO-2004043898 discloses the batch-wise mononitration of alkanediols with stabilized nitric acid in a water-immiscible chlorinated organic solvent at a reaction temperature lower than room temperature (RT), such as preferably equal to or lower than 0°C. The stabilized nitric acid consists of fuming nitric acid diluted in water to a concentration of about 83 to 85% w/w and is substantially free from nitrous acid and nitrogen oxides. The weight ratio of "stabilised" nitric acid to the alkanediols ranges from about 10:1 to about 15:1.

FR1357403 discloses a specific process for the nitration of polyvalent alcohols encompassing inter alia a two-phase reaction mixture.

Next to the use of a chlorinated solvent, which is highly unwanted in chemical production, a significant drawback of this process is the requirement of high acid equivalents which leads to a high salt load. Furthermore, the process uses a non-commercial nitric acid dilution. Also, the reaction requires low temperatures (i.e. < RT) to control the selectivity and to avoid thermal runaways or even explosions.

Thus, there is an ongoing need to develop an efficient, economic and safe industrial process for the direct nitration of α,ω-C₃₋₁₀alkanediol such as in particular of 1,3-propanediol, which can be carried out batch-wise and in the absence of an organic solvent, requires low amounts of nitric acid equivalents and is readily amenable to scale-up.

Surprisingly and in contrast to the common knowledge in the art, it has now been found that ω-nitrooxyC₃₋₁₀alkane-1-ols such as in particular 3-nitrooxypropanol can also be obtained in an economical and safe manner in a batch-wise, 'solvent-free', one-pot process at elevated temperatures when using nitric acid in low amounts as nitration agent.

Thus, the present invention relates to a process for the preparation of ω-nitrooxyC₃₋₁₀alkane-1-ols comprising the nitration of an α,ω-C₃₋₁₀alkanediol with a nitration agent, characterized in that the nitration agent is a mixture consisting of 50 to 75 wt.-%, preferably 50 to 72 wt.-% of nitric acid in water and wherein the nitric acid is used in an amount selected in the range from 0.5 to 5 mol equivalents based on the α,ω-C₃₋₁₀alkanediol, and wherein the nitration is carried out at a temperature selected in the range from 65 - 100°C (nitration temperature).

The term 'α,ω-C₃₋₁₀alkanediols' as used herein refers to the linear α,ω-alkanediols having 3 to 10 carbon atoms such as 1,3-propanediol, 1,4-butanediol, 1,5-pentanediol, 1,6-hexanediol, 1,7-heptanediol, 1,8-octanediol, 1,9-nonanediol and 1,10-decanediol. Particularly preferred in all embodiments according to the present invention is 1,3-propanediol.

The term 'ω-nitrooxyC₃₋₁₀alkane-1-ols' refers to the linear ω-nitrooxyalkane-1-ols having 3 to 10 carbon atoms such as 3-nitrooxypropane-1-ol (also referred to as 3-nitrooxypropanol), 4-nitrooxybutane-1-ol, 5-nitrooxypentane-1-ol, 6-nitrooxyhexane-1-ol, 7-nitrooxyheptane-1-ol, 8-nitrooxyoctane-1-ol, 9-nitrooxynonane-1-ol and 10-nitrooxydecane-1-ol. Particularly preferred in all embodiments according to the present invention is 3-nitrooxypropanol.

In all embodiments of the present invention, preferably 1 to 4 mol equivalents, more preferably 1 to 3 mol equivalents, most preferably 1.5 to 2.5 mol equivalents of nitric acid based on the α,ω-C₃₋₁₀alkanediol such as in particular of 1,3-propanediol is used. Further advantageous ranges include 1 to 2, 1 to 1.5, 1.5 to 5, 2 to 5, 3 to 5 and 2 to 4 mol-equivalents.

In all embodiments of the present invention, the concentration of the nitric acid in the nitric acid water mixture is preferably selected in the range from 60 to 72 wt.-%, even more preferably in the range from 62 to 68 wt.-%. Such nitric acid is readily commercially available (e.g. at Lonza or Yara). Alternatively, regenerated nitric acid can be used.

It is well understood that in all embodiments of the present invention nitric acid is used as sole nitration agent, i.e. in the absence of any sulfuric acid or agents leading to the formation of an activated nitrate (e.g. acetyl nitrate) such as acetic anhydride.

In an advantageous embodiment, the reaction is carried out in the presence of an agent able to remove nitrous acid and nitrogen oxides (i.e. in the presence of a suitable nitrite trapping agent) such as in particular in the presence of urea and/ or sulfamic acid, most preferably in the presence of urea.

In all embodiments of the present invention, the amount of the agent able to remove nitrous acid and nitrogen oxides (in the following called 'nitrite trapping agent') is preferably selected in the range from 0.1 to 0.7 mol equivalents, preferably in the range from 0.2 to 0.5 mol-equivalents, most preferably in the range from 0.2 to 0.4 mol-equivalents, based on the amount of the α,ω-C₃₋₁₀alkanediol such as in particular based on the amount of the 1,3-propanediol.

Furthermore, in all embodiments of the present invention, the nitration is preferably carried out in the absence (i.e. without the addition) of any organic solvent but carried out solely in water, i.e. the nitration is carried out in a reaction mixture consisting essentially of nitric acid, water, the α,ω-C₃₋₁₀alkanediol and optionally the nitrite trapping agent. Preferably, in all embodiments of the present invention a nitrite trapping agent is also present.

The term 'organic solvent' as used herein is well known to a person skilled in the art and refers to carbon-based solvents which are capable of dissolving other substances and which are generally liquid at room temperature. Typical organic solvents encompass aliphatic-chain compounds e.g. hexane, halogenated hydrocarbons e.g. dichloromethane, esters e.g. ethyl acetate, ethers e.g. diethyl ether, aromatic hydrocarbons e.g. toluene or xylene, alcohols e.g. methanol or ethanol as well as ketones e.g. acetone without being limited thereto.

In a particular advantageous embodiment, the present invention relates to a process for the preparation of ω-nitrooxyC₃₋₁₀alkane-1-ols comprising the nitration of an α,ω-C₃₋₁₀alkanediol with a nitration agent in a reaction mixture consisting essentially of nitric acid, water, an α,ω-C₃₋₁₀alkanediol and a nitrite trapping agent, characterized in that the nitration agent is a mixture consisting of 50 to 75 wt.-% of nitric acid in water wherein the nitric acid is used in an amount selected in the range from 0.5 to 5 mol equivalents based on the α,ω-C₃₋₁₀alkanediol, and wherein the nitration is carried out at a temperature selected in the range from 65 - 100°C, with the proviso that all the preferences and definitions as given herein also apply to said process.

Advantageously, in all embodiments of the present invention, the reaction temperature during the nitration (nitration temperature) is selected in the range from 70 to 100°C, preferably in the range from 75 to 100°C, more preferably in the range from 80-100 C, most preferably in the range from 85 to 100°C as this leads to particularly good results, in particular when using 2 to 3 mol equivalents of nitric acid based on the amount of the α,ω-C₃₋₁₀alkanediol.

In one advantageous embodiment, the nitration temperature is selected in the range from about 65 to 75 °C, when 4 to 5 mol equivalents of nitric acid based on the amount of the α,ω-C₃₋₁₀alkanediol, in particular based on the amount of 1,3-propanediol, are used. In another advantageous embodiment, the nitration temperature is advantageously selected in the range from 75 to 100°C, preferably in the range from 80 to 100°C, most preferably in the range from 85 to 100°C, such as from 90 to 100°C, when less than 4 mole equivalents of nitric acid based on the amount of the α,ω-C₃₋₁₀alkanediol, in particular based on the amount of 1,3-propanediol, are used as such process conditions lead to a minimization of undesired by-products (in particular dinitration) while still providing good yields allowing an economical industrial scale production.

The nitration of the α,ω-C₃₋₁₀alkanediol, preferably of 1,3-propanediol is preferably carried out for reaction times ranging from about 30 to about 300 minutes, preferably from about 60 to about 120 minutes, most preferably from 60 to 100 minutes.

The progress of the reaction can be monitored with conventional analytical methods and the optimal reaction time can thus be determined. Thus, in a preferred embodiment, the present invention relates to a process according to the present invention, wherein the reaction is monitored and the reaction is terminated at an in-process yield in the range from 40 to 65 %, preferably in the range from 40 to 55 %, most preferably in the range from 40 to 55 %, such as in the range of 45 to 55 % as determined by HPLC or GC, e.g. by RP-HPLC based on an external standard.

It is well understood, that the process according to the present invention was designed to be carried out batchwise (not continuously) in a safe and economical manner, thus in a preferred embodiment, the present invention is directed to processes for the batchwise nitration of ω-nitrooxyC₃₋₁₀alkane-1-ols with all the definitions and preferences as given herein. Nevertheless, the process can of course also be easily adapted by a person skilled in the art to be carried out continuously.

In a particular advantageous embodiment, the process according to the present invention with all the definitions and preferences as given herein is a process (I) comprising the following consecutive steps:
i. Provision of the nitration agent (e.g. by loading of the reactor vessel with the nitric acid water mixture), followed by
ii. Addition of the agent able to remove nitrous acid and nitrogen oxides (i.e. the nitrite trapping agent) and stirring of the obtained reaction mixture, preferably for 10 to 60 minutes, more preferably for 15 to 45 minutes, followed by
iii. Addition of the α, ω-C₃₋₁₀alkanediol, preferably the 1,3-propanediol (actual nitration reaction)

In a particular advantageous embodiment, the process according to the present invention is a process (II) which is a process (I) comprising next to the consecutive steps (i) to (iii), furthermore the subsequent step of
iv. Quenching of the reaction mixture obtained in (iii) with water and/ or a base to obtain an aqueous reaction mixture comprising the ω-nitrooxyC₃₋₁₀alkane-1-ol, preferably the 3-nitrooxypropanol.

Optionally, the aqueous reaction mixture obtained in (iv) may be further neutralized with a suitable base, in particular in case of quenching with water (i.e. the process contains a subsequent neutralization step (v)).

Suitable bases in all embodiments of the present invention include alkali or earth alkali bases such as alkali or earth alkali hydroxides or carbonates as well as amines without being limited thereto. The bases can be added as such or as an aqueous solution thereof.

Preferably in all embodiments of the present invention the base used in step (iv) and in the optional neutralization step (v) is selected from the group of NaOH, KOH, Ca(OH)₂ or ammonia, more preferably an aqueous solution thereof is used. Most preferably in all embodiments of the present invention the base is aqueous NaOH or ammonia (NH₄OH).

In case of quenching with water preferably a neutralization step (v) is included into the process (II).

The temperature in step (i) and the starting temperature in step (ii) may already be the nitration temperature. It is however preferred that the temperature in step (i) and the starting temperature in step (ii) is selected in the range from 18-30°C, such as at ambient temperature (generally about 20-22°C) and the reaction mixture obtained in (ii) is subsequently heated up to the desired nitration temperature with all the preferences and definitions as given herein prior to the addition of the α,ω-C₃₋₁₀alkanediol, preferably of the 1,3-propanediol (i.e. before step (iii)).

In a further advantageous embodiment, the quenching of the reaction (step (iv)) is performed either with cold water, such as with water having a temperature selected in the range from 0 to 20°C, more preferably in the range from 5 to 15 °C or with an aqueous base such as in particular aqueous NaOH, KOH, Ca(OH)₂ or NH₄OH having a temperature selected in the range from (-20) to 15°C, more preferably in the range from (-10) to 10°C.

In a particular advantageous embodiment, the pH of the aqueous reaction mixture comprising the ω-nitrooxyC₃₋₁₀alkane-1-ol such as in particular 3-nitrooxypropanol in the quenching respectively neutralization step is adjusted to a pH of 8-12, more preferably to a pH of 9 to 11, most preferably with aqueous sodium hydroxide (NaOH), potassium hydroxide (KOH) or aqueous ammonia (NH₄OH).

Preferably, in all embodiments of the present invention, during work-up, the ω-nitrooxyalkane-1-ol, more preferably the 3-nitrooxypropanol, is separated from residual starting material (i.e. the α, ω-C₃₋₁₀alkanediol, preferably the 1,3-propanediol) and from the by-product (i.e. α,ω-C₃₋₁₀alkanedioldinitrate, preferably the 1,3-propanediol dinitrate) by selective extraction.

Thus, the present invention also relates to a process (III) which is a process (I) or (II), which comprises an additional extraction step (vi) either after step (iii), step (iv) or step (v).

Particularly preferred in all embodiments of the present invention is a process (IV) comprising a selective extraction step (vi) after the quenching step (iv) respectively the neutralization step (v).

The pH of the aqueous mixture comprising the ω-nitrooxyC₃₋₁₀alkane-1-ol such as in particular 3-nitrooxypropanol prior to the selective extraction is preferably adjusted to a pH of 8-12, more preferably to a pH of 9 to 11, most preferably with aqueous sodium hydroxide (NaOH), potassium hydroxide (KOH) or aqueous ammonia (NH₄OH).

The selective extraction according to the present invention comprises the extraction of the aqueous reaction mixture obtained in step (iii), (iv) or (v) comprising the ω-nitrooxyC₃₋₁₀alkane-1-ol, preferably the 3-nitrooxypropanol, first with an apolar solvent, such as preferably an aliphatic or aromatic hydrocarbon solvent, more preferably an aromatic solvent such as xylene or toluene, most preferably toluene (to remove impurities! by-products such as e.g. the dinitrated α,ω-C₃₋₁₀alkanediols) followed by extraction with an ether solvent (e.g. a cyclic ether such as tetrahydrofuran or dioxane or an alkyl ether such as diethyl ether, dibutyl ether, diisobutyl ether or methyl tert.-butylether) such as in particular methyl tert-butylether (MtBE) (to extract the ω-nitrooxyC₃₋₁₀alkane-1-ol such as in particular the 3-nitrooxypropanol).

It is furthermore preferred to wash the obtained ether phase with water to ensure complete removal of the α,ω-C₃₋₁₀alkanediol, preferably the 1,3-propanediol. Following evaporation of the ether solvent (optionally after a drying step with e.g. with MgSO₄), pure ω-nitrooxyC₃₋₁₀alkane-1-ol such as in particular the pure 3-nitrooxypropanol is obtained. Even more preferably, the apolar solvent used in the selective extraction such as in particular the hydrocarbon, even more in particular the aromatic hydrocarbon solvent phase is re-extracted with an aqueous solvent, such as e.g. the aqueous solution obtainable from washing the ether phase to further increase the yield of the ω-nitrooxyC₃₋₁₀alkane-1-ol.

The reaction of the process according to the invention can in principle be carried out in any reactor suitable for the respective reaction type. Without restricting generality, the following are mentioned by way of example: suspension reactor, stirred tank, stirred tank cascade, tubular reactor, tubular reactors containing mixing elements like static mixers, shell-type reactor, shell and tube reactor, reactive distillation column.

The aqueous reaction mixture obtained in step (iv) or (v) as outlined above may be supplemented directly to ruminants such as in particular cattle due to economic reasons.

Such aqueous solutions of the 'ω-nitrooxyC₃₋₁₀alkane-1-ol, preferably the 3-nitrooxypropanol can be used to supplement ruminants such as in particular cattle to reduce methane formation in said ruminants. Such aqueous solutions are characterized by a high stability, a low toxicity and an increased cost efficiency as the ω-nitrooxyC₃₋₁₀alkane-1-ol does not need to be isolated before being supplemented to the ruminant.

Such aqueous solutions (all wt.-% are all based on the total weight of the aqueous solution) consist essentially of
(a) 1 to 30 wt.-%, preferably 1 to 15 wt.-%, more preferably 2 to 10 wt.-%, most preferably 5 to 9 wt.-% of a α,ω-C₃₋₁₀alkanediol, preferably of 1,3-propandiol,
(b) 2.5 to 40 wt.-%, preferably 5 to 30 wt.-%, more preferably 5 to 20 wt.-%, most preferably 7.5 to 20 wt.-% of a ω-nitrooxyC₃₋₁₀alkane-1-ol, preferably of 3-nitrooxypropanol,
(c) 0.1 to 5 wt.-%, preferably 0.3 to 3 wt.-%, most preferably 0.5 to 2 wt.-% of a α,ω -dinitrooxyC₃₋₁₀alkane, preferably of 1,3 dinitrooxypropane,
(d) 0.1 to 10 wt.-%, preferably 0.1 to 6 wt.-%, more preferably 0.3 to 4 wt.-%, most preferably 0.5 to 2 wt.-% of a nitrite trapping agent, preferably of sulfamic acid or urea,
(e) 5 to 30 wt.-%, preferably 10 to 25 wt.-%, most preferably 15 to 20 wt.-% of a nitrate, preferably sodium nitrate,
(f) 0.1 to 5 wt.-%, preferably 0.3 to 3 wt.-%, most preferably 0.5 to 2 wt.-% of a base, preferably sodium hydroxide, and
(g) 30 to 80 wt.-%, preferably 40 to 70 wt.-%, most preferably 50 to 65 wt.-% of water.

Preferred aqueous solutions (all wt.-% are all based on the total weight of the aqueous solution) according to the present invention consist essentially of
(a) 5 to 10 wt.-% of 1,3-propanediol,
(b) 5 to 20 wt.-% of 3-nitrooxypropanol,
(c) 0.5 to 3 wt.-% of 1,3 dinitrooxypropane,
(d) 0.5 to 4 wt.-% of urea,
(e) 15 to 20 wt.-% of sodium nitrate,
(f) 0.5 to 3 wt.-% of sodium hydroxide, and
(g) 50 to 65 wt.-% of water.

Most preferred aqueous solutions (all wt.-% are all based on the total weight of the aqueous solution) according to the present invention consist essentially of
(a) 5 to 9 wt.-% of 1,3-propandiol,
(b) 5 to 20 wt.-% of 3-nitrooxypropanol,
(c) 0.5 to 2 wt.-% of 1,3-dinitrooxypropane,
(d) 0.5 to 2 wt.-% of urea,
(e) 15 to 20 wt.-% of sodium nitrate,
(f) 0.5 to 2 wt.-% of sodium hydroxide, and
(g) 50 to 65 wt.-% of water.

The term 'consist essentially of' as used according to the present invention means that the amounts of the ingredients (a) to (g) sum up to 100 wt.-%. It is, however, not excluded that small amount of impurities or additives may be present which are, for example, introduced via the respective raw materials of the ingredients (a) to (g).

The following examples are used to further illustrate the invention without being limited thereto.

### Example 1: Preparation of 3-nitrooxypropanol (3-NOP)

### A ) Nitrate ester formation

The nitration was carried out in a double-jacket reactor that was kept open, preferably with a light nitrogen flow above the reaction during the whole nitration process. Nitric acid (1.5 to 4 equivalents based on the 1,3-propanediol as indicated in table 1) was loaded into the reactor and stirred for 15 min at 20°C. Urea (0.3 mol eq) was added and stirring was continued for 30 min. Meanwhile, the jacket temperature was set to the reaction temperature (65 to 100 °C). Then 1,3-propanediol (1 mol eq) was dosed into the acid during 15 min via a dropping funnel and the reaction was stirred for 60-100 min. Then the reaction was quenched by pouring it onto water cooled to 10-15 °C in an amount to dilute the nitric acid to ~20%. The quenched reaction solution was slowly adjusted to pH 9-11 with 30 % aq. NaOH. Alternatively, the reaction was quenched by pouring it onto 20 % aqueous NaOH cooled to (-10) to 10 °C such as to obtain an aqueous solution of pH 9-11.

### B) Work-up (Selective Extraction)

The aqueous solution obtained in step A were subsequently extracted with toluene followed by methyl tert-butyl ether (MtBE). The MtBE-phases were washed with water and evaporated to dryness to obtain 3-nitrooxypropanol (1,3-propanediol mononitrate) as a colorless to slightly yellow oil.

**Table 1**

| **#** | **eq (HNO₃)** | **c (HNO₃) [% w/w]** | **T [°C]** | **Reaction time [min]** | **in-process-yield^{#} [%]** | |
|---|---|---|---|---|---|---|
| 1 | 4.5 | 65 | 65 | 90 | | 55 |
| 2 | 4 | 65 | 70 | 60 | | 54 |
| 3 | 4 | 60 | 90 | 45 | | 54 |
| 4 | 3 | 65 | 80 | 60 | | 54 |
| 5 | 2 | 65 | 80 | 90 | | 38 |
| 6 | 2 | 65 | 90 | 90 | | 42 |
| 7 | 1.5 | 65 | 100 | 90 | | 41 |
| 8 (Ref) | 2 | 65 | 60 | 120 | | 21 |
| 9 (Ref) | 14 | 68 | 20 | 60 | tends to be instable | |
| 10 (Ref) | 4 | 85 | 60 | 90 | 18* (isolated yieid°) | |
| 11 (Ref) | 14 | 85 | 0 | 90 | | 0.1* |

| | | | | | | |
|---|---|---|---|---|---|---|
| *extensive dinitration, reaction slightly instable during 1,3-propanediol addition ^{#}isolated yield ~ 1-10% lower compared to in-process yield | | | | | | |

Synthesis of 3-nitrooxypropanol (PDMN) as outlined above using the conditions in entry Table 1, entry 5 (4 eq. 65% HNOs, 70 °C, 60 min) with a quench onto 20 % NaOH resulted in 52 % yield on a 25 g scale.

Synthesis of 3-nitrooxypropanol (PDMN) as outlined above using the conditions in entry 6 with a quench onto 20 % NaOH resulted in 38 % isolated yield on a 50 g scale.

### C.) Composition of the neutralized reaction mixture

### (conditions: Table 1, entry 2, quench onto 20 % NaOH)

| **Compound** | **wt.-%** |
|---|---|
| 1,3-Propandiol | 2.5 |
| 3-Nitrooxypropanol | 5.2 |
| 1,3-dinitrooxypropane | 2.2 |
| Urea | 1.6 |
| Sodium hydroxide | 0.1 |
| Sodium nitrate | 24.2 |
| Water | Ad 100 |

### Example 2

Synthesis of propanediol mononitrate (PDMN) as outlined above (4 eq. 65% HNOs, 60 °C, 90 min) with 0.3 eq. sulfamic acid instead of urea resulted in 34 % isolated yield.

### Example 3

The example as outlined above (entry 1: 4 eq 65% HNO₃, 60 °C, 90 min) was repeated with 1,4-butanediol instead of 1,3-propanediol resulting in 19% isolated yield of butanediol mononitrate.

## Claims

1. A process for the preparation of ω-nitrooxyC₃₋₁₀alkane-1-ols comprising the nitration of an α,ω-C₃₋₁₀alkanediol with a nitration agent, optionally in the presence of a nitrite trapping agent, **characterized in that** the nitration agent is a mixture consisting of 50 to 75 wt.-% of nitric acid in water wherein the nitric acid is used in an amount selected in the range from 0.5 to 5 mol equivalents based on the α,ω-C₃₋₁₀alkanediol, and wherein the nitration is carried out at a temperature selected in the range from 65 - 100°C.

2. The process according to claim 1, **characterized in that** the ω-nitrooxyC₃₋₁₀alkane-1-ol is 3-nitrooxypropanol and the α,ω-C₃₋₁₀alkanediol is 1,3-propanediol.

3. The process according to claim 1 or 2, wherein 1 to 4 mol equivalents, preferably 1 to 3 mol equivalents of nitric acid, based on the amount of the α,ω-C₃₋₁₀alkanediol is used.

4. The process according to any one or more of claims 1 to 3, wherein the concentration of the nitric acid in water is selected in the range from 60 to 72 wt.-%, preferably in the range from 62 to 68 wt.-%.

5. The process according to any one or more of claims 1 to 4, wherein the nitrite trapping is sulfamic acid and/ or urea, most preferably urea.

6. The process according to any one or more of claims 1 to 5, wherein the amount of the nitrite trapping agents is selected in the range from 0.1 to 0.7 mol equivalents, preferably in the range from 0.2 to 0.4 mol-equivalents, based on the α, ω-C₃₋₁₀alkanediol.

7. The process according to any one or more of claims 1 to 6, wherein the reaction temperature is selected in the range from 70 to 100°C, more preferably in the range from 75 to 100°C, most preferably in the range from 80 to 100°C.

8. The process according to any one or more of claims 1 to 7, wherein the nitration is carried out for a time ranging from 10 to 300 minutes.

9. The process according to anyone of claims 1 to 8 comprising the following steps:
i. Provision of the nitration agent, followed by
ii. Addition of the nitrite trapping agent and preferably stirring of the obtained reaction mixture for 10 to 60 minutes, more preferably for 15 to 45 minutes, followed by
iii. Addition of the α, ω-C₃₋₁₀alkanediol, preferably the 1,3-propanediol and optionally
iv. Quenching of the reaction mixture obtained in (iii) with water or a base to obtain an aqueous reaction mixture comprising the ω-nitrooxyC₃₋₁₀alkane-1-ol.

10. The process according to claim 9, wherein the aqueous reaction mixture, optionally after neutralization with a suitable base, is consecutively extracted with first an aromatic hydrocarbon solvent and second an ether solvent.

11. The process according to claim 9 or 10, wherein the aromatic hydrocarbon solvent is toluene and the ether solvent is methyl tert-butylether.

## Patentansprüche

1. Verfahren zur Herstellung von ω-Nitrooxy-C₃₋₁₀-alkan-1-olen, umfassend die Nitrierung eines α, ω-C₃₋₁₀-Alkandiols mit einem Nitrierungsmittel, gegebenenfalls in Gegenwart eines Nitrit-Abfangmittels, **dadurch gekennzeichnet, dass** es sich bei dem Nitrierungsmittel um eine Mischung handelt, die aus 50 bis 75 Gew.-% Salpetersäure in Wasser besteht, wobei die Salpetersäure in einer Menge im Bereich von 0,5 bis 5 Moläquivalenten, bezogen auf das α, ω-C₃₋₁₀-Alkandiol, verwendet wird und wobei die Nitrierung bei einer Temperatur im Bereich von 65-100 °C durchgeführt wird.

2. Verfahren nach Anspruch 1, **dadurch gekennzeichnet, dass** es sich bei dem ω-Nitrooxy-C₃₋₁₀-alkan-1-ol um 3-Nitrooxypropanol handelt und es sich bei dem α, ω-C₃₋₁₀-Alkandiol um 1,3-Propandiol handelt.

3. Verfahren nach Anspruch 1 oder 2, wobei 1 bis 4 Moläquivalente, vorzugsweise 1 bis 3 Moläquivalente, Salpetersäure, bezogen auf das α, ω-C₃₋₁₀-Alkandiol, verwendet werden.

4. Verfahren nach einem oder mehreren der Ansprüche 1 bis 3, wobei die Konzentration der Salpetersäure in Wasser im Bereich von 60 bis 72 Gew.-%, vorzugsweise im Bereich von 62 bis 68 Gew.-%, gewählt wird.

5. Verfahren nach einem oder mehreren der Ansprüche 1 bis 4, wobei es sich bei dem Nitrit-Abfangmittel um Sulfamidsäure und/oder Harnstoff, ganz besonders bevorzugt Harnstoff, handelt.

6. Verfahren nach einem oder mehreren der Ansprüche 1 bis 5, wobei die Menge der Nitrit-Abfangmittel im Bereich von 0,1 bis 0,7 Moläquivalenten, vorzugsweise im Bereich von 0,2 bis 0,4 Moläquivalenten, bezogen auf das α, ω-C₃₋₁₀-Alkandiol, gewählt wird.

7. Verfahren nach einem oder mehreren der Ansprüche 1 bis 6, wobei die Reaktionstemperatur im Bereich von 70 bis 100 °C, weiter bevorzugt im Bereich von 75 bis 100 °C, ganz besonders bevorzugt im Bereich von 80 bis 100 °C, gewählt wird.

8. Verfahren nach einem oder mehreren der Ansprüche 1 bis 7, wobei die Nitrierung über einen Zeitraum im Bereich von 10 bis 300 Minuten durchgeführt wird.

9. Verfahren nach einem der Ansprüche 1 bis 8, dass die folgenden Schritte umfasst:
i. Bereitstellen des Nitrierungsmittels, gefolgt von
ii. Zugabe des Nitrit-Abfangmittels und vorzugsweise Rühren der erhaltenen Reaktionsmischung über einen Zeitraum von 10 bis 60 Minuten, weiter bevorzugt 15 bis 45 Minuten, gefolgt von
iii. Zugabe des α, ω-C₃₋₁₀-Alkandiols, vorzugsweise des 1,3-Propandiols, und gegebenenfalls
iv. Quenchen der in (iii) erhaltenen Reaktionsmischung mit Wasser oder einer Base unter Erhalt einer wässrigen Reaktionsmischung, die das ω-Nitrooxy-C₃₋₁₀-alkan-1-ol umfasst.

10. Verfahren nach Anspruch 9, wobei die wässrige Reaktionsmischung, gegebenenfalls nach Neutralisation mit einer geeigneten Base, nacheinander als Erstes mit einem aromatischen Kohlenwasserstoff-Lösungsmittel und als Zweites mit einem Ether-Lösungsmittel extrahiert wird.

11. Verfahren nach Anspruch 9 oder 10, wobei es sich bei dem aromatischen Kohlenwasserstoff-Lösungsmittel um Toluol handelt und es sich bei dem Ether-Lösungsmittel um Methyl-tert-butylether handelt.

## Revendications

1. Procédé pour la préparation de ω-nitrooxyC₃₋₁₀alcan-1-ols comprenant la nitration d'un α, ω-C₃₋₁₀alcanediol avec un agent de nitration, éventuellement en la présence d'un agent de piégeage de nitrite, **caractérisé en ce que** l'agent de nitration est un mélange constitué de 50 à 75 % en poids d'acide nitrique dans l'eau, l'acide nitrique étant utilisé en une quantité choisie dans la plage de 0,5 à 5 équivalents en moles sur la base du α, ω-C₃₋₁₀alcanediol, et, la nitration étant mise en œuvre à une température choisie dans la plage de 65 à 100 °C.

2. Procédé selon la revendication 1, **caractérisé en ce que** le ω-nitrooxyC₃₋₁₀alcan-1-ol est le 3-nitrooxypropanol et le α, ω-C₃₋₁₀alcanediol est le 1,3-propanediol.

3. Procédé selon la revendication 1 ou 2, 1 à 4 équivalents en moles, préférablement 1 à 3 équivalents en moles d'acide nitrique, sur la base de la quantité du α, ω-C₃₋₁₀alcanediol, étant utilisés.

4. Procédé selon l'une quelconque ou plusieurs des revendications 1 à 3, la concentration de l'acide nitrique dans l'eau étant choisie dans la plage de 60 à 72 % en poids, préférablement dans la plage de 62 à 68 % en poids.

5. Procédé selon l'une quelconque ou plusieurs des revendications 1 à 4, le piégeage de nitrite étant l'acide sulfamique et/ou l'urée, le plus préférablement l'urée.

6. Procédé selon l'une quelconque ou plusieurs des revendications 1 à 5, la quantité des agents de piégeage de nitrite étant choisie dans la plage de 0,1 à 0,7 équivalent en moles, préférablement dans la plage de 0,2 à 0,4 équivalent en moles, sur la base du α,ω-C₃₋₁₀alcanediol.

7. Procédé selon l'une quelconque ou plusieurs des revendications 1 à 6, la température de réaction étant choisie dans la plage de 70 à 100 °C, plus préférablement dans la plage de 75 à 100 °C, le plus préférablement dans la plage de 80 à 100 °C.

8. Procédé selon l'une quelconque ou plusieurs des revendications 1 à 7, la nitration étant mise en œuvre pendant une durée dans la plage de 10 à 300 minutes.

9. Procédé selon l'une quelconque des revendications 1 à 8 comprenant les étapes suivantes :
i. fourniture de l'agent de nitration, suivie par
ii. ajout de l'agent de piégeage de nitrite et préférablement agitation du mélange réactionnel obtenu pendant 10 à 60 minutes, plus préférablement pendant 15 à 45 minutes, suivis par
iii. ajout du α, ω-C₃₋₁₀alcanediol, préférablement du 1,3-propanediol et éventuellement
iv. extinction du mélange réactionnel obtenu en (iii) avec de l'eau ou une base pour obtenir un mélange réactionnel aqueux comprenant le ω-nitrooxyC₃₋₁₀alcan-1-ol.

10. Procédé selon la revendication 9, le mélange réactionnel aqueux, éventuellement après neutralisation avec une base appropriée, étant consécutivement extrait avec premièrement un solvant hydrocarboné aromatique et deuxièmement un solvant de type éther.

11. Procédé selon la revendication 9 ou 10, le solvant hydrocarboné aromatique étant le toluène et le solvant de type éther étant l'éther de méthyle et de tert-butyle.
